# EUROPEAN PATENT SPECIFICATION

(11) **EP 0 934 261 B1**
(45) Date of publication and mention of the grant of the patent: **31.10.2001**
(21) Application number: 97942102.1
(22) Date of filing: 23.09.1997
(51) Int. Cl.: C07C 319/04, C07C 319/14, C07C 319/24, C07C 323/66, C07F 9/38

(54) **PROCESS FOR PRODUCING DITHIOBIS-ALKANESULFONATES AND PHOSPHONATES**
VERFAHREN ZUR HERSTELLUNG VON DITHIOBIS-ALKANESULFONATEN UND PHOSPHONATEN
PROCEDE DE PRODUCTION DE DITHIOBIS-SULFONATES ET DE PHOSPHONATES

(30) Priority: 01.10.1996 US 28212 P
(43) Date of publication of application: 11.08.1999
(73) Proprietor: BIONUMERIK PHARMACEUTICALS, INC., San Antonio, Texas 78229 (US)
(72) Inventor: HARIDAS, Kochat, San Antonio, TX 78248 (US)
(74) Representative: Lucas, Brian Ronald
(86) International application number: PCT/GB97/02576
(87) International publication number: WO 98/14426

(56) References cited:
- US-A- 3 639 430
- US-A- 5 347 015
- CHEMICAL ABSTRACTS, vol. 108, no. 22, 30 May 1988 Columbus, Ohio, US; abstract no. 193285b, H. LEE, ET AL.: "Adsorbtion of ordered zirconium phosphonate multilayer films on silicon and gold surfaces" page 432; XP002050051 & JOURNAL OF PHYSICAL CHEMISTRY, vol. 92, no. 9, 1988, pages 2597-2601,
- CHEMICAL ABSTRACTS, vol. 110, no. 3, 16 January 1989 Columbus, Ohio, US; abstract no. 23982a, J.T. DOI, ET AL.: "Neighbouring group participation in organic redox reactions. 13. Intramolecular interaction of the beta-phosphonic acid group in the aqueous iodine oxidation of thio ethers and disulphides. Generation of a phosphonic-phosphoric anhydride" page 563; XP002050052 & PHOSPHORUS SULFUR, vol. 35, no. 1-2, 1988, pages 173-182,
- E. BRZEZINSKA, ET AL.: "Disulphides. 1. Syntheses using 2,2'-dithiobis(benzothiazole)" JOURNAL OF ORGANIC CHEMISTRY, vol. 59, no. 26, 30 December 1994, WASHINGTON, DC, US, pages 8239-8244, XP002050050

## Description

This invention relates to a process for producing dithiobis-alkanesulfonates and phosphonates especially disodium 2,2'-(dithiobis)ethane sulfonate (dimesna; NaSO₃CH₂CH₂-S-S-CH₂CH₂SO₃Na).

### BACKGROUND OF THE INVENTION

Compounds of the general formula:

(I): R₂₋(CH₂)ₘ-S-S-(CH₂)ₘ-R₂

wherein R₂ is SO₃M or PO₃M₂ wherein M or each M independently is sodium, potassium or hydrogen and m is 2, 3 or 4, are useful *inter alia* as chemotherapeutic protective agents used to mitigate the toxicity of platinum complex antitumor drugs which are given to patients with certain types of cancer. Thus, dimesna can be co-administered with cisplatin (cis-diamminedichloroplatinum) to protect the body against nephrotoxicity, and with carboplatin (cisdiammine-1,1-cyclobutanedicarboxylatoplatinum) to protect the body against myelosuppression.

The chief prior processes for synthesizing dimesna (and like disulfides) include the conversion of various alkanesulfonic acids into their respective mercaptan derivatives (such as mesna) and the subsequent oxidation of the mercaptans into their respective disulfides (such as dimesna) by use of iodine-containing reagents, such as iodate. These processes, while efficient, required isolation procedures to be performed to isolate and purify the end products from the reagents used. These processes generated environmental pollutants, which required disposal and could not be carried out in a single reaction vessel.

E. Brzezinska *et al.*, *J.* Org. Chem. 1994, *59,* 8239-8244, have described reacting various thiols, including mesna, with 2,2'-dithiobis(benzothiazole) to produce corresponding disulfides, including dimesna.

### SUMMARY OF THE INVENTION

The present invention avoids these disadvantages in the production of dimesna and provides a more convenient method of making dithiobis-alkanesulfonates and phosphonates.

The invention provides a process of producing compounds of the general formula:

R₂(CH₂)ₘ-S-S-(CH₂)ₘ-R₂ (I);

wherein R₂ is SO₃M or PO₃M₂ wherein M is sodium, potassium or hydrogen;
and m is 2, 3 or 4,
which comprises heating a mercaptan of formula:

R₂-(CH₂)ₘ-SH (III)

wherein m is as defined above,
with oxygen gas under pressure.

The invention further provides a preferred process of making compounds of the general formula I, said process comprising
(1) reacting a compound of formula

   CH₂X-CHY-(CH₂)ₙ-R₂ (II),

   wherein
   X and Y together complete an olefinic carbon-carbon double bond or, where R₂ is PO₃M₂, X can be halo and Y is then hydrogen;
   n is 0, 1 or 2; and
   R₂ is as defined above, with a sulfide of the general formula Z-SH, wherein Z is hydrogen, sodium or potassium, provided that where R₂ is PO₃M_{2,} the reaction is carried out in the presence of a free radical initiator when X and Y together represent a said double bond or with the aid of heat when X represents halo and Y is hydrogen;
   to form a mercaptan of formula HS-(CH₂)ₘ-R₂; wherein R₂ and m are as defined above, and
(2) heating the mercaptan produced in Step (1) with oxygen gas, under pressure, to produce the compound of formula (I).

The preferred process is summarised by the following chart:

CH₂ = CH - (CH₂)ₙ - R₂ (IIa)

or

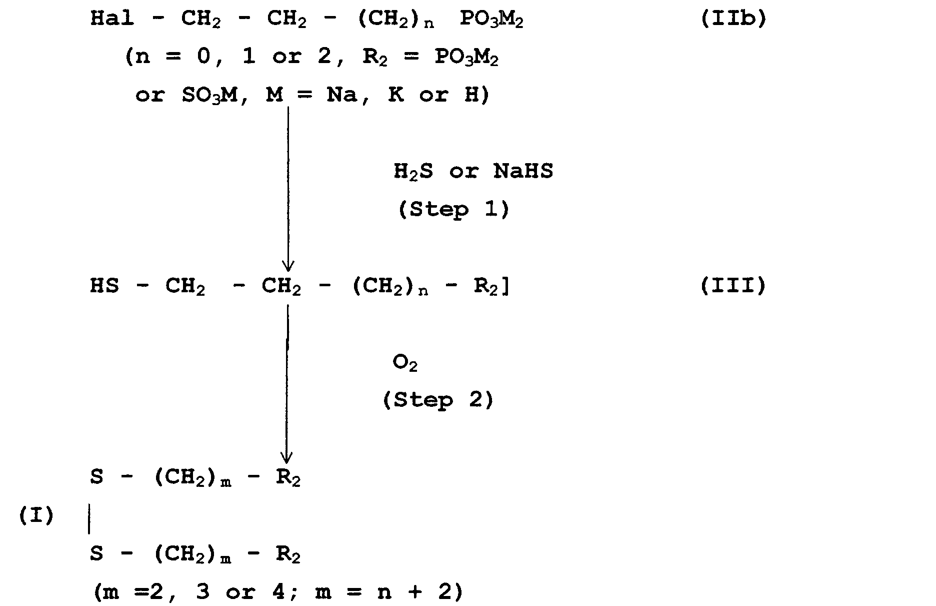

### DESCRIPTION OF THE PREFERRED EMBODIMENTS

The preferred process of this invention for preparing the compounds of formula I involves two steps in a single-pot, which results in the conversion of an alkenyl sulfonate salt or acid (^{ω} -alkenesulfonate or - sulfonic acid) to the desired formula I compound, especially dimesna which can be produced thereby in a highly pure form, on a large scale.

Step 1 involves the addition of a sulfhydryl moiety in an anti-Markovnikov fashion to the unsaturated terminal double bond by generating an sp³ center. The addition to the double bond is effected by reacting the starting alkenyl sulfonate salt with a hydrosulfide salt or with hydrogen sulfide, preferably in a slightly basic solution (pH from 8 to 9.5). The sulfide is preferably present in at least a stoichiometric proportion and usually in a molar excess of at least 2:1, preferably from 3:1 to 5:1. This step forms a mercapto-(alternatively termed a sulfhydryl-) alkanesulfonate.

Step 2 of this process involves the oxidization of the mercaptoalkanesulfonate to a disulfide and is performed in an aqueous medium and in the same reaction vessel as step 1, without the need to purify or isolate the product of step 1. Step 2 includes the introduction of oxygen gas, preferably by bubbling, into the reaction vessel, along with an increase in pressure and temperature above ambient values, preferably at a slightly basic pH. The preferred pH is from 8 to 9.5. It can remain unadjusted from step 1 which is a big advantage. The preferred temperature is at least 40°, most preferably at least 60°C. A range of 40 to 100°C is contemplated for most purposes. The preferred gauge (superatmospheric) pressure is at least 20psi (138 kPa), more preferably at least 30psi (207 kPa) and most desirably at least 50psi (345kPa). A range of 20 to 60psi (138 to 414kPa) is contemplated for most purposes. Dimesna or a homologue or analogue thereof can be formed in substantially quantitative yield. The desired final product can be easily crystallized from the aqueous reaction medium itself.

When a phosphonate of formula I is desired, the starting compound can be a haloalkanephosphonate, preferably a bromoalkane- or chloroalkanephosphonate. Preferably n is O or 1, the starting material then being a haloethane- or halopropanephosphonate. The two step, single pot process involves first the treatment of this starting compound with sodium hydrosulfide or hydrogen sulfide at elevated temperature, especially from 40 to 120°. The sulfide is preferably used in molar excess, as described above. Alternatively, step 1 may be achieved by converting the alkenephosphonic acid to the mercaptan by addition of a sulfur source, conditions and reagents being as described above, in the presence of a free radical initiator. Step 2, the oxidation to the disulfide, is the same as described above.

The following non-limiting examples illustrate the invention.

### EXAMPLE 1

### Disodium 2,2' - (dithiobis)ethanesulfonate

100mL of a 25% aqueous stock solution (25 grams VSA, 0.192 mole) of vinylsulfonic acid (VSA) sodium salt (Aldrich Chemical Company) was taken up in a Parr vessel, and argon gas bubbled in for one hour to deoxygenate the aqueous solution. To this solution was added 33.5 grams (0.598 mole, reckoned as NaSH) of sodium hydrosulfide monohydrate (Aldrich Chemical Company) and 10mL of sodium hydroxide. The pH of the solution was approximately 9.0. The reaction mixture was agitated in a Parr apparatus for two hours, during which time NMR monitoring was conducted at 30 minute intervals.

The product obtained from this step was taken to the next step without isolation, heated to 60°C, and oxygen bubbled into the vessel for thirty minutes. The vessel was then pressurized to 50psi (345kPa) gauge and agitated for six more hours at 60°C.

The completed reaction mixture was then worked up by concentrating the aqueous fraction at 80°C using an industrial vacuum, followed by diffused recrystallization from water. The crystallized product was then lyophilized after adjusting the pH to 7.2 by adding IN HCl and filtering through a 0.2 micrometre pore membrane filter. NMR and elemental analysis confirmed the presence of pure (99%) disodium 2,2'-(dithiobis)ethanesulfonate.

### EXAMPLE 2

### Tetrasodium 2,2' - (dithiobis)ethanephosphonate

2-Chloroethanephosphonic acid (1 gram; 6.9 mmoles) was taken up in anhydrous ethanol (10ml) and degassed with a continuous stream of argon for at least 30 minutes. This was then added to a boiling solution of sodium hydrosulfide hydrate (1.4 g, 25 mmol, reckoned as NaSH) in ethanol to obtain a reaction mixture with a final pH of approximately 9. The resultant reaction mixture was then refluxed for 10 hours. The reaction mixture was then cooled and the pH adjusted to 8 using 1N HC1. The solvent was removed and the product was purified by diffused crystallization. The white solid was then taken into a Parr bottle and 50 ml water added. The aqueous solution was then bubbled with a stream of oxygen for a period of at least one hour. Then the bottle was pressurized with 50psi (345kPa) gauge oxygen and shaken at 60°C for 4 hours. The product was isolated by concentrating the aqueous portion to half at 80°C under industrial vacuum, followed by crystallization. The product thus obtained was then characterized by high field NMR and elemental analysis and by comparing with an authentic sample.

### EXAMPLE 3

### Tetrasodium 2,2' - (dithiobis)ethanephosphonate

Example 2 was repeated except that the same molar amount of 2-bromoethanephosphonic acid was used as the starting material and the ethanol solvent replaced by water. The title compound thus obtained was then characterized by high field NMR and elemental analysis and by comparing with an authentic sample.

## Claims

1. A process of producing compounds of the general formula:
R₂-(CH₂)ₘ-S-S-(CH₂)ₘ-R₂ (I)
wherein R₂ is SO₃M or PO₃M₂ wherein M is sodium,
potassium or hydrogen;
and m is 2, 3 or 4,
which comprises heating a mercaptan of formula:
R₂- (CH₂)ₘ-SH (III)
wherein m is as defined above,
with oxygen gas under pressure.

2. A process according to Claim 1, wherein the pressure is at least 20 psi (138 kPa) gauge and the reaction is carried out at a temperature of at least 60°C.

3. A process according to Claim 2, wherein the pressure is at least 30 psi (207 kPa) gauge.

4. A process according to Claim 1, 2 or 3 wherein R₂ is SO₃M and m is 2.

5. A process according to Claim 4, wherein M is sodium and the product is dimesna.

6. A process for producing compounds of the general formula:
R₂-(CH₂)ₘ-S-S-(CH₂)ₘ-R₂ (I);
wherein R₂ is SO₃M or PO₃M₂ wherein M is sodium,
potassium or hydrogen and m is 2, 3 or 4,
which comprises:
(1) reacting a compound of formula
CH₂X-CHY- (CH₂)ₙ-R₂ (II);
wherein X and Y together complete an olefinic carbon-carbon double bond, or, where R₂ is PO₃M₂, X can be halo and Y is then hydrogen;
n is 0, 1 or 2; and
R₂ is as defined as above.
with a sulfide of the general formula Z-SH, wherein Z is hydrogen, sodium or potassium; provided that where R₂ is PO₃M₂, the reaction is carried out in the presence of a free radical initiator when X and Y together complete a said double bond or with the aid of heat when X represents halo and Y is hydrogen;
to form a mercaptan of formula HS-(CH₂)ₘ-R₂;
wherein R₂ and m are as defined above; and
(2) heating the mercaptan produced in step (I) with oxygen gas under pressure to produce the compound of formula (I).

7. A process according to Claim 6, wherein in step (2), the pressure is at least 20 psi (138 kPa) gauge and the reaction is carried out at a temperature of at least 60°C.

8. A process according Claim 6 or 7, wherein R₂ is SO₃M and m is 2.

9. A process according to Claim 8, wherein the starting compound is a vinyl sulfonate of formula II wherein X and Y together form a double bond, n is O and R₂ is SO₃M, the mercaptan recation product of step (I) is not isolated and step (2) is carried out in the same reaction vessel as for step (I).

10. A process according to Claim 8 or 9, wherein M is sodium and the product is dimesna.

## Patentansprüche

1. Verfahren zur Herstellung von Verbindungen der allgemeinen Formel:
R₂₋(CH₂)ₘ-S-S-(CH₂)ₘ-R₂ (I)
worin R₂ SO₃M oder PO₃M₂ ist, worin M Natrium, Kalium oder Wasserstoff ist,
und m 2, 3 oder 4 ist,
das das Erwärmen eines Mercaptans der Formel:
R₂-(CH₂)ₘ-SH (III)
worin m wie vorstehend definiert ist,
mit Sauerstoffgas unter Druck umfasst.

2. Verfahren nach Anspruch 1. wobei der Druck mindestens 20 psi (138 kPa), Manometer, beträgt und die Reaktion bei einer Temperatur von mindestens 60°C erfolgt.

3. Verfahren nach Anspruch 2, wobei der Druck mindestens 30 psi (207 kPa), Manometer, beträgt.

4. Verfahren nach Anspruch 1, 2 oder 3, wobei R₂ SO₃M ist und m 2 beträgt.

5. Verfahren nach Anspruch 4, wobei M Natrium ist und das Produkt Dimesna ist.

6. Verfahren zur Herstellung von Verbindungen der allgemeinen Formel:
R₂-(CH₂)ₘ-S-S-(CH₂)ₘ-R₂ (I)
worin R₂ SO₃M oder PO₃M₂ ist, worin M Natrium, Kalium oder Wasserstoff ist, und m 2, 3 oder 4 ist,
welches umfasst:
(1) Reaktion einer Verbindung der Formel
CH₂X-CHY-(CH₂)ₙ-R₂ (II)
worin X und Y zusammen eine olefinische Kohlenstoff-Kohlenstoff-Doppelbindung vervollständigen oder, falls R₂ PO₃M₂ ist, X ein Halogenatom sein kann und Y dann Wasserstoff ist,
n 0, 1 oder 2 ist und
R₂ wie vorstehend definiert ist,
mit einem Sulfid der allgemeinen Formel Z-SH, worin Z Wasserstoff, Natrium oder Kalium ist, vorausgesetzt, dass, falls R₂ PO₃M₂ ist, die Reaktion in Gegenwart eines Radikalbildners, wenn X und Y zusammen die Doppelbindung vervollständigen, oder mit Hilfe von Wärme erfolgt, wenn X ein Halogenatom ist und Y Wasserstoff ist,
wodurch ein Mercaptan der Formel HS-(CH₂)ₘ-R₂ erzeugt wird,
worin R₂ und m wie vorstehend definiert sind, und
(2) Erwärmen des im Schritt (I) erzeugten Mercaptans mit Sauerstoffgas unter Druck, wodurch die Verbindung der Formel (I) erzeugt wird.

7. Verfahren nach Anspruch 6, wobei im Schritt (2) der Druck mindestens 20 psi (138 kPa), Manometer, beträgt und die Reaktion bei einer Temperatur von mindestens 60°C erfolgt.

8. Verfahren nach Anspruch 6 oder 7, wobei R₂ SO₃M ist und m 2 beträgt.

9. Verfahren nach Anspruch 8, wobei die Ausgangsverbindung ein Vinylsulfonat der Formel II ist, worin X und Y zusammen eine Doppelbindung bilden, n 0 ist und R₂ SO₃M ist, wobei das Mercaptanreaktionsprodukt vom Schritt (I) nicht abgetrennt wird und der Schritt (2) im gleichen Reaktionsgefäß wie beim Schritt (I) erfolgt.

10. Verfahren nach Anspruch 8 oder 9, wobei M Natrium ist und das Produkt Dimesna ist.

## Revendications

1. Procédé de préparation de composés de formule générale :
R₂-(CH₂)ₘ-S-S-(CH₂)ₘ-R₂ (I)
dans laquelle R₂ représente SO₃M ou PO₃M₂, où M représente un atome de sodium, de potassium ou d'hydrogène ;
et m vaut 2, 3 ou 4,
qui comprend l'étape consistant à chauffer un mercaptan de formule :
R₂-(CH₂)ₘ-SH (III)
dans laquelle m répond à la même définition que ci-dessus,
avec de l'oxygène gazeux sous pression.

2. Procédé selon la revendication 1, dans lequel la pression manométrique est d'au moins 20 psi (138 kPa) et la réaction est réalisée à une température d'au moins 60°C.

3. Procédé selon la revendication 2, dans lequel la pression manométrique est d'au moins 30 psi (207 kPa).

4. Procédé selon la revendication 1, 2 ou 3, dans lequel R₂ représente SO₃M et m vaut 2.

5. Procédé selon la revendication 4, dans lequel M représente un atome de sodium et le produit est le dimesna.

6. Procédé de préparation de composés de formule générale :
R₂-(CH₂)ₘ-S-S-(CH₂)ₘ-R₂ (II);
dans laquelle R₂ représente SO₃M ou PO₃M₂, où M représente un atome de sodium, de potassium ou d'hydrogène, et m vaut 2, 3 ou 4, qui comprend les étapes consistant :
(1) à faire réagir un composé de formule
CH₂X-CHY-(CH₂)ₙ-R₂ (II);
dans laquelle X et Y complètent conjointement une double liaison oléfinique carbone-carbone ou dans laquelle, lorsque R₂ représente PO₃M₂, X peut être un atome d'halogène et Y est alors un atome d'hydrogène ;
n vaut 0, 1 ou 2 ; et
R₂ répond à la même définition que ci-dessus,
avec un sulfure de formule générale Z-SH, dans laquelle Z représente un atome d'hydrogène, de sodium ou de potassium ; étant entendu que lorsque R₂ représente PO₃M₂, la réaction est réalisée en présence d'un amorceur à radicaux libres lorsque X et Y complètent conjointement ladite double liaison, ou à l'aide de chauffage lorsque X représente un atome d'halogène et Y un atome d'hydrogène ;
pour former un mercaptan de formule HS-(CH₂)ₘ-R₂ ;
dans laquelle R₂ et m répondent aux mêmes définitions que ci-dessus ; et
(2) à faire chauffer le mercaptan produit dans l'étape (I) avec de l'oxygène gazeux sous pression pour produire le composé de formule (I).

7. Procédé selon la revendication 6, dans l'étape (2) duquel la pression manométrique est d'au moins 20 psi (138 kPa) et la réaction est réalisée à une température d'au moins 60°C.

8. Procédé selon la revendication 6 ou 7, dans lequel R₂ représente SO₃M et m vaut 2.

9. Procédé selon la revendication 8, dans lequel le composé de départ est un vinylsulfonate de formule II où X et Y forment conjointement une double liaison, n vaut O et R₂ représente SO₃M, le mercaptan produit dans l'étape (I) n'est pas isolé et l'étape (2) est réalisée dans le même récipient réactionnel que l'étape (I).

10. Procédé selon la revendication 8 ou 9, dans lequel M représente un atome de sodium et le produit est le dimesna.
